# EUROPÄISCHE PATENTSCHRIFT

(11) **EP 2 895 590 B1**
(45) Veröffentlichungstag und Bekanntmachung des Hinweises auf die Patenterteilung: **15.06.2016**
(21) Anmeldenummer: 13737150.6
(22) Anmeldetag: 16.07.2013
(51) Int. Cl.: C12M 1/00, B01D 46/00, C12M 1/12

(54) **KOMBIFILTER**
COMBI-FILTER
FILTRE COMBINÉ

(30) Priorität: 12.09.2012 DE 102012017972
(43) Veröffentlichungstag der Anmeldung: 22.07.2015
(73) Patentinhaber: Sartorius Stedim Biotech GmbH, 37079 Göttingen (DE)
(72) Erfinder: REIF, Oscar-Werner, 30173 Hannover (DE); GRELLER, Gerhard, 37085 Göttingen (DE); NIKOLOUDIS, Paschalis, 37079 Göttingen (DE); LAUSCH, Ralf, 37079 Göttingen (DE); HUSEMANN, Ute, 37079 Göttingen (DE); LOEWE, Thomas, 37077 Göttingen (DE); FRIESE, Thomas, 37083 Göttingen (DE); DREHER, Thomas, 37083 Göttingen (DE)
(74) Vertreter: Stehl, Astrid
(86) Internationale Anmeldenummer: PCT/EP2013/002103
(87) Internationale Veröffentlichungsnummer: WO 2014/040669

(56) Entgegenhaltungen:
- DE-A1-102008 025 968
- JP-A- H05 161 827
- US-A1- 2003 041 572

## Beschreibung

### Gebiet der Erfindung

Die Erfindung betrifft eine Vorrichtung zur Kultivierung von Zellen in einem Bioreaktor, dessen aus dem Reaktorinnenraum herausführende Abluftleitung einen Abluftfilter mit einer steril filtrierenden Mikrofiltermembran aufweist.

### Stand der Technik

Zur Kultivierung von Zellen in einem Bioreaktor muss dem Reaktorinnenraum, der eine geschlossenen Kultivierungskammer bildet, ein relativ großer Luftstrom sicher zu- und abgeführt werden. Zurückgehalten werden müssen dabei Zellen (z. B. Mikroorganismen) sowohl von außen nach innen als auch von innen nach außen. Der Reaktorinnenraum bzw. die Kultivierungskammer wird in der Regel unter erhöhter Temperatur mit einer belüfteten wässrigen Lösung betrieben, welche Nährstoffe und Zellen (z.B. Mikroorganismen) enthält, welche vermehrt werden sollen.

Es werden nach dem Stand der Technik hydrophobe steril filtrierende Mikrofiltermembranen eingesetzt, die beispielsweise nach ASTM 838-05 qualifiziert sind.

Vorzugsweise sind diese so hydrophob, dass sowohl kein Wasser in die Filtermembranstruktur eindringt als auch sich kein geschlossener Wasserfilm auf der Filtermembranoberfläche bilden und so den Luftstrom durch die Filtermembran einschränken bzw. stoppen kann. Wird Wasserdampf mit dem Luftstrom aus der Lösung ausgetragen, so kondensiert dieser mindestens teilweise an der Filtermembran und allen Gehäuse- und Leitungsteilen und muss abgeführt werden. Im Besonderen bei hohen Luftströmen werden Zellen, Zellreste oder partikuläre Stoffe, wie z.B. Nährstoffe oder Reste davon, mit auf die Filtermembranoberfläche getragen und verblocken die Porenstruktur der Filtermembran.

Aus der DE 10 2008 025 968 A1 ist eine Vorrichtung zur Kultivierung von Zellen in einem Bioreaktor bekannt, dessen aus dem Reaktorinnenraum herausführende Abluftleitung einen hydrophoben Steril- oder Abluftfilter mit einer Mikrofiltermembran aufweist. Um das Kondensieren von Wasserdampf an der Filtermembran zu vermeiden, wird bei dem bekannten Bioreaktor dem Abluftfilter ein Kondensator vorgelagert, der warme feuchte Abluft herunterkühlt und abtropfendes Wasser in den Reaktorinnenraum zurückführt. Dies hat sich zwar grundsätzlich bewährt, ist jedoch relativ kostenintensiv und kann nicht verhindern, dass Zellen, Zellreste oder partikuläre Stoffe auf die Filtermembranoberfläche getragen werden und diese verblocken.

US 2003/004 1572 A1 offenbart einen Kombinationsfilter für die Luftfiltration in Inkubatoren. Der Kombinationsfilter umfasst einen HEPA-Filter in Kombination mit einem VOC-Filter.

### Aufgabenstellung

Es ist die Aufgabe der vorliegenden Erfindung, die bekannte Vorrichtung zur Kultivierung von Zellen so zu verbessern, dass sie einerseits kostengünstig hergestellt werden kann und dass sie andererseits neben der Vermeidung einer Verblockung durch Wasserdampf auch eine Verblockung der Mikrofiltermembran durch Zellen oder partikuläre Stoffe verhindert.

### Darlegung der Erfindung

Diese Aufgabe wird in Verbindung mit den Merkmalen des Oberbegriffs von Anspruch 1 dadurch gelöst, dass dem Abluftfilter zum Reaktorinnenraum hin mindestens ein Vorfilter mit einem hydrophoben Filtermaterial vorgelagert ist.

Durch die Verwendung eines hydrophoben Filtermaterials als Vorfilter wird der steril filtrierende Mikrofilter einfach und kostengünstig vor einem unerwünschten Verblocken sowohl durch Wasserdampf oder Wasser als auch durch Zellen, Zellreste oder partikuläre Stoffe geschützt.

Bevorzugte Ausführungsformen sind Gegenstand der abhängigen Ansprüche.

Gemäß einer bevorzugten Ausführungsform der Erfindung bilden der Vorfilter und der Abluftfilter einen gemeinsamen Kombinationsfilter und sind insbesondere in einem gemeinsamen Gehäuse angeordnet. Dabei sind der Vorfilter und der Abluftfilter als zwei getrennte hintereinander in dem Gehäuse angeordnete Filtereinsätze ausgebildet. Es ist aber auch möglich, das Filtermaterial des Vorfilters direkt auf der zu schützenden Mikrofiltermembran des Abluftfilters anzuordnen. So kann der Vorfilter beispielsweise koaxial zum Abluftfilter in dem Gehäuse angeordnet werden.

Grundsätzlich ist es auch möglich, den Kombinationsfilter in eine Zuflussleitung zum Bioreaktor einzusetzen.

Gemäß der Erfindung ist das Filtermaterial als ein Glasfaserfilter ausgebildet. Es ist aber auch möglich, das Filtermaterial des Vorfilters aus Melt-Blown-Faserfiltermaterialien aus PE (Polyethylen), PP (Polypropylen), PET (Polyethylenterephthalat), PESU (Polyethersulfon), PVDF (Polyvinylidenfluorid) oder PMP (Polymethylpenten) auszubilden. Unter Melt-Blown versteht der Fachmann einen Prozess, in dem nichtgewebte Stoffe bzw. Vliese direkt aus Granulat hergestellt werden. Dabei wird ein spezielles Spinnverfahren in Kombination mit Hochgeschwindigkeits-Heißluft genutzt, um feinfaserige Stoffe mit unterschiedlichen Strukturen zu produzieren.

Nach der Erfindung weist das Filtermaterial des Vorfilters eine durchschnittliche Porengröße von größer als 0,1 µm und kleiner als 100 µm auf. Weiter bevorzugt kann die durchschnittliche Porengröße zwischen 3 µm und 30 µm sein. Insbesondere kann die durchschnittliche Porengröße zwischen 10 µm und 15 µm sein.

Gemäß einer weiteren bevorzugten Ausführungsform der Erfindung weist das Filtermaterial des Vorfilters eine Dicke von 50 µm bis 250 µm auf.

Nach einer weiteren bevorzugten Ausführungsform der Erfindung weist der Abluftfilter eine hydrophobe Mikrofiltermembran aus PVDF (Polyvinylidenfluorid), ePTFE (expandiertes Polytetrafluorethylen), PP oder PE auf.

Gemäß einer weiteren bevorzugten Ausführungsform der Erfindung weist der Abluftfilter eine hydrophobe Mikrofiltermembran auf.

Die hydrophobe Mikrofiltermembran kann dabei aus einem hydrophoben polymeren Material gebildet sein, wobei die Membranmatrix und ihre inneren und äußeren Oberflächen jeweils hydrophob sind. Die vorgenannten polymeren Materialien sind bevorzugt aus der Gruppe umfassend PVDF, PTFE (Polytetrafluorethylen), PMP, PP, PE oder Kombinationen davon ausgewählt. Bei einer weiteren bevorzugten Ausführungsform ist die Membranmatrix hydrophil, während ihre inneren und äußeren Oberflächen durch Beschichtung oder Pfropfpolymerisation hydrophobe Eigenschaften aufweisen.

Gemäß einer weiteren bevorzugten Ausführungsform der Erfindung weist der Abluftfilter eine hydrophobe Mikrofiltermembran mit Porengrößen von größer als 0,1 µm, bevorzugt 0,2 µm, auf.

Weitere Merkmale und Vorteile der Erfindung ergeben sich aus der nachfolgenden speziellen Beschreibung und den Zeichnungen.

### Kurzbeschreibung der Zeichnungen

Es zeigen:
- Figur 1:: eine schematische Darstellung einer Vorrichtung zur Kultivierung von Zellen in einem Bioreaktor mit in getrennten Gehäusen angeordneten Abluftfilter und Vorfilter,
- Figur 2:: eine schematische Darstellung einer Vorrichtung zur Kultivierung von Zellen in einem Bioreaktor mit Abluftfilter und Vorfilter, die in einem gemeinsamen Gehäuse als Kombinationsfilter angeordnet sind,
- Figur 3:: eine schematische Darstellung einer Vorrichtung zur Kultivierung von Zellen in einem Bioreaktor mit Abluftfilter und Vorfilter, die in einem gemeinsamen Gehäuse als Kombinationsfilter angeordnet sind, wobei das Filtermaterial des Vorfilters direkt auf der zu schützenden Mikrofiltermembran des Abluftfilters aufliegt,
- Figur 4:: eine Seitenansicht im Schnitt und Ausriss einer weiteren Vorrichtung entsprechend Figur 1 und
- Figur 5:: eine Seitenansicht im Schnitt und Ausriss einer weiteren Vorrichtung entsprechend Figur 3, bei der das Filtermaterial des Vorfilters die Mikrofiltermembran des Abluftfilters konzentrisch umschließt.

### Beschreibung bevorzugter Ausführungsformen

Eine Vorrichtung 1 zur Kultivierung von Zellen besteht im Wesentlichen aus einem Bioreaktor 2, einer Abluftleitung 3, einem Abluftfilter 4 und einem Vorfilter 5.

Der Bioreaktor 2 weist einen Reaktorinnenraum 6 auf, der über die Abluftleitung 3 mit dem Abluftfilter 4 verbunden ist. Dem Abluftfilter 4 ist zum Reaktorinnenraum 6 hin der Vorfilter 5 vorgelagert.

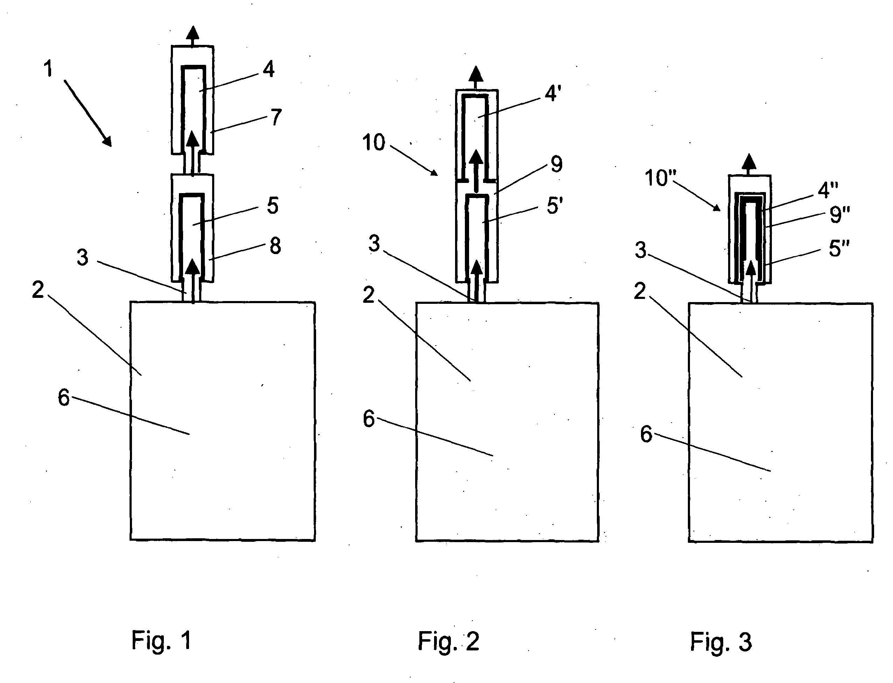

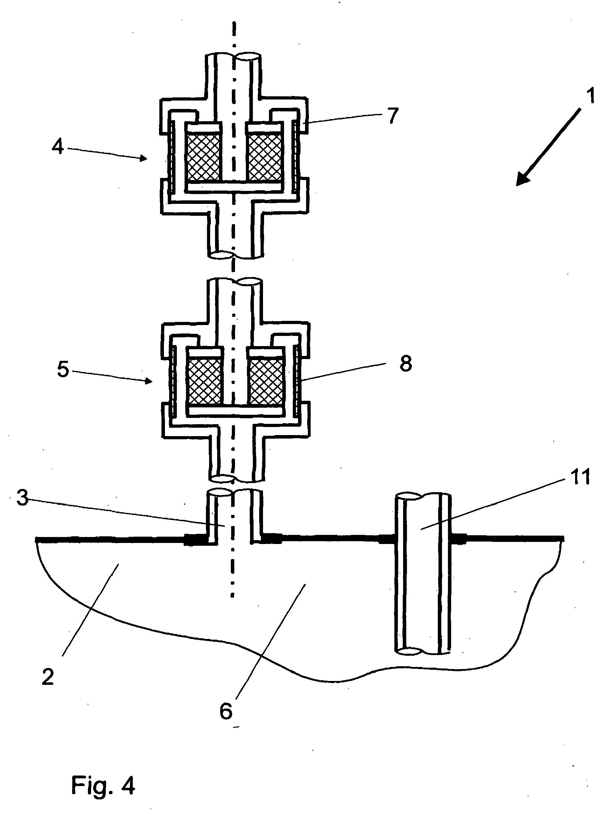

Entsprechend den Figuren 1 und 4 ist der Abluftfilter 4 in einem Abluftfiltergehäuse 7 und der Vorfilter 5 in einem Vorfiltergehäuse 8 angeordnet.

Entsprechend der Figur 2 sind der Abluftfilter 4' und der Vorfilter 5' in einem gemeinsamen Filtergehäuse 9 angeordnet und bilden einen gemeinsamen Kombinationsfilter 10.

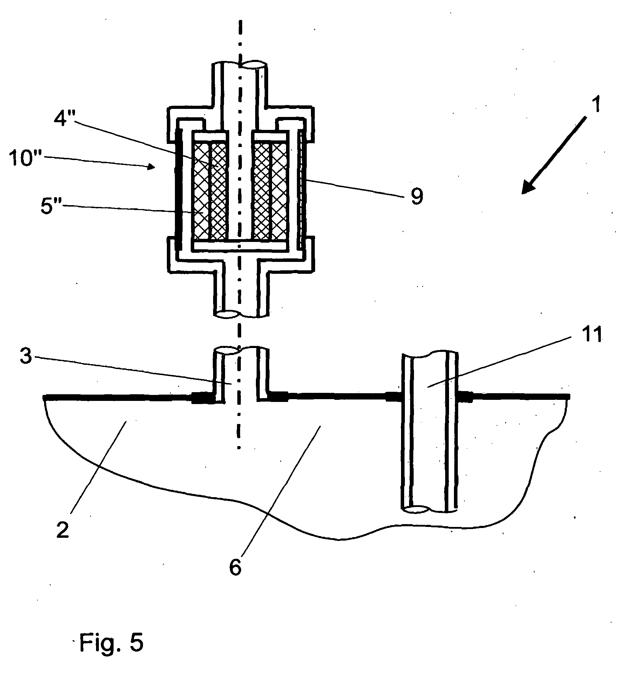

Entsprechend den Figuren 3 und 5 ist der Vorfilter 5" konzentrisch zum Abluftfilter 4" angeordnet, so dass der Vorfilter 5" den Abluftfilter 4" abdeckt. Der Kombinationsfilter 10" ist mit seinem Vorfilter 5" und seinem Abluftfilter 4" in dem gemeinsamen Filtergehäuse 9" angeordnet.

Die Vorfilter 5, 5', 5" weisen eine durchschnittliche Porengröße zwischen 10 und 15 µm auf. Das Filtermaterial der Vorfilter 5, 5', 5" weist eine Dicke von 50 bis 250 µm auf. Die hydrophobe Mikrofiltermembran des Abluftfilters 4, 4', 4" weist im Ausführungsbeispiel eine Porengröße von 0,2 µm auf.

Der Bioreaktor 2 weist zu seinem Reaktorinnenraum 6 hin einen Gaszuführkanal 11 auf, über den Gas zugeführt werden kann.

Die nachfolgenden Ausführungsbeispiele wurden erfolgreich getestet:

### Entsprechend dem Ausführungsbeispiel von Fig. 1:

Vorfilter 5: eine Lage 10-15 µm Vorfiltermaterial plissierte Kerze/Capsule 1: BH9 Capsule, 1 Lage PP Melt Blown Filtermaterial, Mean Flow Porengröße 10-15 µm (Topas), Dicke 150 µm, Basisgewicht 20 g/m²
Abluftfilter 4: Sartofluor Sterilfilter, Plissierte Kerze/Capsule 2: BH9 Capsule, 1 Lage ePTFE Mikrofiltermembran.0,2 µm

### Entsprechend dem Ausführungsbeispiel der Figuren 3 und 5 Kombifilter 10" (1 Lage 10-15 µm Vorfiltermaterial vor 1 Lage ePTFE Mikrofiltermembran 0,2 µm):

Vorfilter 5'': Plissierte Kerze/Capsule: BH9 Capsule, 1 Lage PP Melt-Blown-Filtermaterial Mean Flow Porengröße 10-15 µm (Topas), Dicke 150 µm, Basisgewicht 20 g/m² (vor Abluftfilter)
Abluftfilter 4": 1 Lage ePTFE Mikrofiltermembran 0,2 µm Bewertung: Funktion gegeben, besonders einfacher Aufbau, kleinstes Baumaß, einfachster Betrieb, identisch zu Betrieb ohne Vorfilter.

Natürlich stellen die in der speziellen Beschreibung diskutierten und in den Figuren gezeigten Ausführungsformen nur illustrative Ausführungsbeispiele der vorliegenden Erfindung dar. Dem Fachmann ist im Lichte der hiesigen Offenbarung ein breites Spektrum von Variationsmöglichkeiten an die Hand gegeben. Insbesondere können die Filter 4, 4', 4", 5, 5', 5" bzw. die Kombinationsfilter 10, 10" auch im Gaszuführkanal 11 zusätzlich angeordnet werden. Auch ist es möglich, dem Kombinationsfilter 10, 10" einen nicht weiter dargestellten Kondensator zum Reaktorinnenraum 6 hin vorzulagern.

### Bezugszeichenliste

- 1: Vorrichtung
- 2: Bioreaktor
- 3: Abluftleitung
- 4, 4', 4": Abluftfilter
- 5, 5', 5": Vorfilter
- 6: Reaktorinnenraum
- 7: Abluftfiltergehäuse
- 8, 8": Vorfiltergehäuse
- 9, 9": Filtergehäuse
- 10, 10": Kombinationsfilter
- 11: Gaszuführkanal

## Patentansprüche

1. Vorrichtung (1) zur Kultivierung von Zellen in einem Bioreaktor (2), dessen aus dem Reaktorinnenraum herausführende Abluftleitung (3) einen Abluftfilter (4, 4', 4") mit einer steril filtrierenden Mikrofiltermembran aufweist,
wobei dem Abluftfilter (4, 4', 4") zum Reaktorinnenraum (6) hin mindestens ein Vorfilter (5, 5', 5") mit einem hydrophoben Filtermaterial vorgelagert ist,
**dadurch gekennzeichnet,**
**dass** das Filtermaterial des Vorfilters (5, 5', 5") als ein Glasfaserfilter oder aus Melt-Blown-Faserfiltermaterialien aus PE, PP, PET, PESU, PVDx oder PMP ausgebildet ist und
**dass** das Filtermaterial des Vorfilters (5, 5', 5") eine durchschnittliche Porengröße von größer als 0,1 µm und kleiner als 100 µm aufweist.

2. Vorrichtung nach Anspruch 1,
**dadurch gekennzeichnet,**
**dass** der Vorfilter (5, 5") und der Abluftfilter (4', 4") einen gemeinsamen Kombinationsfilter (10, 10") bilden.

3. Vorrichtung nach Anspruch 1 oder 2,
**dadurch gekennzeichnet,**
**dass** das Filtermaterial des Vorfilters (5") direkt auf der zu schützenden Mikrofiltermembran des Abluftfilters (4") aufliegt.

4. Vorrichtung nach Anspruch 1,
**dadurch gekennzeichnet,**
**dass** das Filtermaterial des Vorfilters (5, 5', 5") eine durchschnittliche Porengröße von größer als 3 µm und kleiner als 30 µm aufweist.

5. Vorrichtung nach Anspruch 4,
**dadurch gekennzeichnet,**
**dass** das Filtermaterial des Vorfilters (5, 5', 5") eine durchschnittliche Porengröße von 10 bis 15 µm aufweist.

6. Vorrichtung nach einem der Ansprüche 1 bis 5,
**dadurch gekennzeichnet,**
**dass** das Filtermaterial des Vorfilters (5, 5', 5") eine Dicke von 50 bis 250 µm aufweist.

7. Vorrichtung nach einem der Ansprüche 1 bis 6,
**dadurch gekennzeichnet,**
**dass** der Abluftfilter (4, 4', 4") eine hydrophobe Mikrofiltermembran aus PVDF, ePTFE, PP oder PE aufweist.

8. Vorrichtung nach einem der Ansprüche 1 bis 6,
**dadurch gekennzeichnet,**
**dass** der Abluftfilter (4, 4', 4") eine hydrophobe Mikrofiltermembran aufweist.

9. Vorrichtung nach einem der Ansprüche 1 bis 8,
**dadurch gekennzeichnet,**
**dass** der Abluftfilter (4, 4', 4") eine hydrophobe Mikrofiltermembran mit Porengröße von größer als 0,1 µm, bevorzugt 0,2 µm aufweist.

## Claims

1. Device (1) for cultivation of cells in a bioreactor (2), the exhaust air line (3) of which leading out of the reactor interior space comprises an exhaust air filter (4, 4', 4") with a sterile filtrating microfilter membrane,
wherein at least one pre-filter (5, 5', 5") with a hydrophobic filter material is mounted upstream of the exhaust air filter (4, 4', 4") with respect to the direction towards the reactor interior space (6),
**characterised in that**
the filter material of the pre-filter (5, 5', 5") is formed as a glass-fibre filter or from melt-blown fibrous filter materials of PE, PP, PET, PESU, PVDx or PMP and
the filter material of the pre-filter (5, 5', 5") has an average pore size of more than 0.1 microns and less than 100 microns.

2. Device according to claim 1, **characterised in that** the pre-filter (5, 5") and the exhaust air filter (4, 4") form a common combination filter (10, 10').

3. Device according to claim 1 or 2, **characterised in that** the filter material of the prefilter (5") rests directly on the microfilter membrane, which is to be protected, of the exhaust air filter (4").

4. Device according to claim 1, **characterised in that** the filter material of the pre-filter (5, 5', 5") has an average pore size of more than 3 microns and less than 30 microns.

5. Device according to claim 4, **characterised in that** the filter material of the pre-filter (5, 5', 5") has an average pore size of 10 to 15 microns.

6. Device according to any one of claims 1 to 5, **characterised in that** the filter material of the pre-filter (5, 5', 5") has a thickness of 50 to 250 microns.

7. Device according to any one of claims 1 to 6, **characterised in that** the exhaust air filter (4, 4', 4") comprises a hydrophobic microfilter membrane of PVDF, ePTFE, PP or PE.

8. Device according to any one of claims 1 to 6, **characterised in that** the exhaust air filter (4, 4', 4") comprises a hydrophobic microfilter membrane.

9. Device according to any one of claims 1 to 8, **characterised in that** the exhaust air filter (4, 4', 4") comprises a hydrophobic microfilter membrane with a pore size of more than 0.1 microns, preferably 0.2 microns.

## Revendications

1. Dispositif (1) pour la culture de cellules dans un bioréacteur (2), dont la conduite d'évacuation d'air (3) partant de l'intérieur du réacteur présente un filtre d'extraction (4, 4', 4") avec une membrane de microfiltration stérile,
au moins un pré-filtre (5, 5', 5") avec un matériau filtrant hydrophobe étant disposé en amont du filtre d'extraction (4, 4', 4") vers l'intérieur du réacteur (6),
**caractérisé en ce que**
le matériau filtrant du pré-filtre (5, 5', 5") est réalisé comme filtre en fibres de verre ou en matériaux à fibre de verre soufflés par fusion constitués de PE, PP, PET, PESU, PVDx ou PMP, et
**en ce que** le matériau filtrant du pré-filtre (5, 5', 5") présente une grandeur de pore moyenne supérieure à 0,1 µm et inférieure à 100 µm.

2. Dispositif selon la revendication 1,
**caractérisé en ce que**
le pré-filtre (5, 5") et le filtre d'extraction (4', 4") forment un filtre combiné (10, 10") commun.

3. Dispositif selon la revendication 1 ou 2,
**caractérisé en ce que**
le matériau filtrant du pré-filtre (5") repose directement sur la membrane de microfiltration du filtre d'extractions (4") à protéger.

4. Dispositif selon la revendication 1,
**caractérisé en ce que**
le matériau filtrant du pré-filtre (5, 5', 5") présente une grandeur de pore moyenne supérieure à 3 µm et inférieure à 30 µm.

5. Dispositif selon la revendication 4,
**caractérisé en ce que**
le matériau filtrant du pré-filtre (5, 5', 5") présente une grandeur de pore moyenne comprise entre 10 et 15 µm.

6. Dispositif selon l'une des revendications 1 à 5,
**caractérisé en ce que**
le matériau filtrant du pré-filtre (5, 5', 5") présente une épaisseur comprise entre 50 et 250 µm.

7. Dispositif selon l'une des revendications 1 à 6,
**caractérisé en ce que**
le filtre d'extraction (4, 4', 4") comporte une membrane de microfiltration hydrophobe en PVDF, ePTFE, PP ou PE.

8. Dispositif selon l'une des revendications 1 à 6,
**caractérisé en ce que**
le filtre d'extraction (4, 4', 4") comporte une membrane de microfiltration hydrophobe.

9. Dispositif selon l'une des revendications 1 à 8,
**caractérisé en ce que**
le filtre d'extraction (4, 4', 4") comporte une membrane de microfiltration hydrophobe ayant une grandeur de pore supérieure à 0,1 µm, préférentiellement à 0,2 µm.
